# EUROPÄISCHE PATENTSCHRIFT

(11) **EP 3 634 570 B1**
(45) Veröffentlichungstag und Bekanntmachung des Hinweises auf die Patenterteilung: **29.11.2023**
(21) Anmeldenummer: 18728093.8
(22) Anmeldetag: 28.05.2018
(51) Int. Cl.: A61N 1/375, H01R 13/02

(54) **IMPLANTIERBARE ELEKTRISCHE VERBINDUNGSSTRUKTUR ZWISCHEN EINEM ELEKTRISCHEN IMPLANTAT UND EINER ELEKTRISCHEN ZU- UND ABLEITUNGSSTRUKTUR**
IMPLANTABLE ELECTRIC CONNECTING STRUCTURE BETWEEN AN ELECTRIC IMPLANT AND AN ELECTRIC FEED AND DRAIN LINE STRUCTURE
STRUCTURE DE CONNEXION ÉLECTRIQUE IMPLANTABLE ENTRE UN IMPLANT ÉLECTRIQUE ET UNE STRUCTURE D'ALIMENTATION ET DE DÉRIVATION ÉLECTRIQUE

(30) Priorität: 09.06.2017 DE 102017209773
(43) Veröffentlichungstag der Anmeldung: 15.04.2020
(73) Patentinhaber: Neuroloop GmbH, 79108 Freiburg (DE)
(72) Erfinder: PLACHTA, Dennis, 79279 Vörstetten (DE); KIMMIG, Fabian, 79108 Freiburg (DE); BORETIUS, Tim, 79108 Freiburg (DE)
(74) Vertreter: Rösler, Uwe
(86) Internationale Anmeldenummer: PCT/EP2018/063864
(87) Internationale Veröffentlichungsnummer: WO 2018/224341

(56) Entgegenhaltungen:
- WO-A1-2016/055512
- US-A- 5 324 322

## Beschreibung

### Technisches Gebiet

Die Erfindung bezieht sich auf eine implantierbare elektrische Verbindungsstruktur zwischen einem elektrischen Implantat, das über wenigstens einen elektrischen Leiter verfügt, und einer elektrischen Zu- und Ableitungsstruktur und auf ein entsprechendes Verfahren zur Herstellung einer solchen Verbindungsstruktur.

### Stand der Technik

Elektronische Implantate, die für einen dauerhaften oder zumindest langfristigen Verbleib im Körper geeignet sind, werden typischerweise zur therapeutischen Beeinflussung von Organfunktionen eingesetzt. Bekannte Beispiele sind Herz- oder Hirnschrittmacher. Gattungsgemäße Implantate verfügen je nach Anwendungszweck und Komplexität der therapeutischen Zielsetzung über eine Vielzahl elektrische Zu- und Ableitungen, über die das jeweilige Implantat mit elektrischen Steuer- bzw. Regelsignalen sowie mit elektrischer Energie versorgt wird. Die Anzahl der elektrischen Zu- bzw. Ableitungen bei elektronisch komplexen Implantaten kann durchaus zwanzig und mehr elektrische Leitungen umfassen, die zu einem flexiblen Kabel zusammengefasst sind, dass das Implantat mit einem Steuergerät, das zumeist mit einer Energiequelle kombiniert ist, verbindet. Die intrakorporale Verortung des Steuergerätes sowie der Energiequelle erfolgt zumeist subkutan in einem Körperbereich, wie beispielsweise im Brustbereich oder nahe des Schlüsselbeins, in dem für die Person möglichst geringe bewegungsbedingte äußere sowie innere Belastungen auftreten und ein leichter operativer Zugang möglich ist.

In der Regel sind die elektrischen Zu- und Ableitungen zwischen dem Implantat und dem Steuergerät bzw. der Energiequelle nicht einstückig ausgebildet, sondern über wenigstens eine Schnittstelle in Form einer intrakorporalen Steckverbindung oder einer lös- oder unlösbaren elektrischen Verbindung, bspw. in Form einer Bond- oder Lötverbindung realisiert. Dies wirft zum einen Probleme auf, die mit dem für die Schnittstelle erforderlichen Bauraum zusammenhängen, darüber hinaus ist es erforderlich, den Schnittstellenbereich aufgrund des feuchten intrakorporalen Milieus entsprechend feuchteresistent auszubilden.

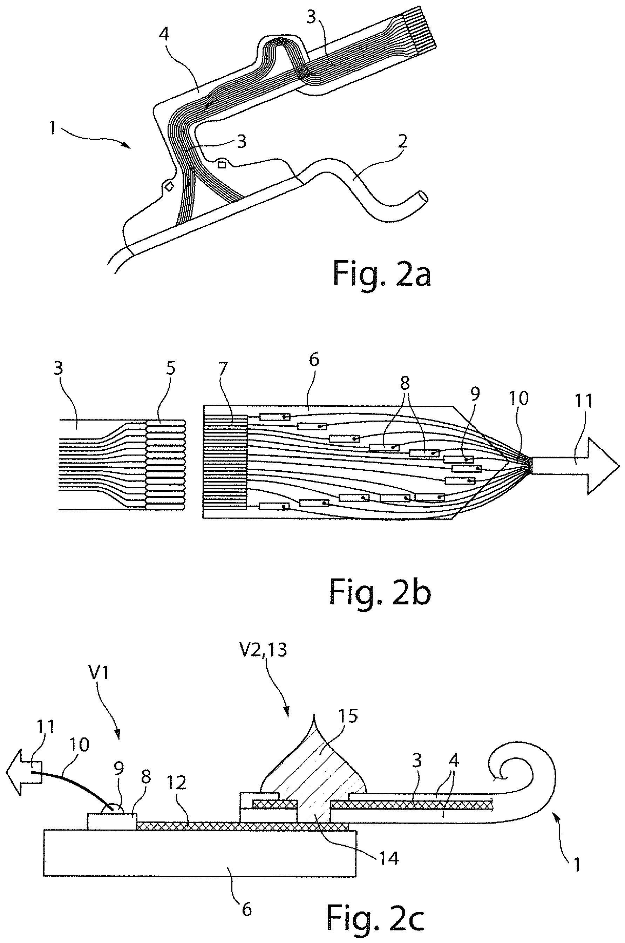

Am Beispiel einer über eine vielpolige Zu- und Ableitungsstruktur zu versorgende, implantierbare Cuff-Elektrodenanordnung, wie sie aus der Druckschrift WO 2016/055512 A1 zu entnehmen ist, soll die bislang bestehende Problematik einer an sich bekannten implantierbaren elektrischen multipoligen Verbindungsstruktur unter Bezugnahme auf die aus den Figuren 2a bis c zu entnehmenden Illustrationen näher erläutert werden,

Figur 2a zeigt ein elektrisches Implantat in Form einer Cuff-Elektrodenanordnung 1, die als Wickelelektrode zur Umfassung um ein Nervenfaserbündel 2 ausgebildet ist. Zu Zwecken einer therapeutischen Stimulation des Nervenfaserbündels 2 sieht die Cuff-Elektrodenanordnung 1 eine Vielzahl einzelne Elektrodenflächen vor, die separat voneinander mit elektrischer Energie sowie Steuersignalen zu versorgen sind. Hierzu verläuft die Vielzahl einzelner elektrischer Zu- und Ableitungen 3 innerhalb eines flexiblen, flächig ausgebildeten Trägersubstrats 4, das als Polymerfolie ausgebildet ist. Die zahlreichen elektrischen Zu- und Ableitungen 3 enden in Form einer stirnseitigen Anschlussstruktur in Form von nebeneinanderliegend angeordneten, so genannten elektrisch leitenden Mikroflexstrukturen 5, die in siehe Figur 2b detailliert dargestellt sind und die es einzeln elektrisch zu kontaktieren gilt.

Zu Zwecken der elektrischen Kontaktierung der Mikroflexstrukturen 5 dient in an sich bekannter Weise eine Keramikadapterplatte 6, auf der entsprechend der Anzahl und Anordnung der Mikroflexstrukturen 5 so genannte Mikroflexkontakte bzw. Mikroflexpads 7 angebracht sind, die in Kontakt mit den Mikroflexstrukturen 5 gebracht werden und elektrisch leitend jeweils einzeln mit an der Oberfläche der Keramikadapterplatte 6 angebrachten Elektrodenflächen 8 verbunden sind. Über Löt- oder Bondverbindungen 9 sind einzelne elektrische Drähte 10 einer elektrischen Zu- und Ableitungsstruktur 11 mit den einzelnen als Lötpads ausgebildeten Elektrodenflächen 8 verbunden.

In Figur 2c ist ein schematisierter Längsschnitt durch die in Figur 2b illustrierte Keramikadapterplatte 6 dargestellt, auf der die elektrischen Verbindungen V1, V2 zwischen den implantatseitigen elektrischen Zu- und Ableitungen 3 einerseits und den in die elektrische Zu- und Ableitungsstruktur 11 mündenden Drähten 10 andererseits detailliert gezeigt sind. Die Drähte 10 sind mittels einer Löt- oder Bondverbindung 9 an auf der Keramikadapterplatte 6 vorgesehenen Elektrodenflächen 8 elektrisch kontaktiert. Die Elektrodenflächen 8 ihrerseits sind einzeln mit auf der Keramikadapterplatte 6 angebrachten, elektrischen Leiterstrukturen 12 verbunden, die vorzugsweise in Form von Platin/Gold-Leiterbahnen ausgebildet sind. Zum Zwecke der elektrischen Verbindung V2 der jeweils implantatseitigen, elektrischen Zu- und Ableitungen 3 mit der auf der Keramikadapterplatte 6 aufgebrachten elektrischen Verbindungsstrukturen 12 dient ein sog. Mikroflexkontakt 13. Hierzu sieht das als Polymerfolie ausgebildete Trägersubstrat 4, innerhalb dem die einzelnen elektrischen Zu- und Ableitungen 3 eingebettet sind, jeweils eine das Trägersubstrat 4 sowie die jeweilige elektrische Zu- und Ableitung 3 durchsetzende Öffnung 14 auf, in die ein sog. Ballbond 15, vorzugsweise bestehend aus Gold, eingebracht ist.

Um den elektrischen sowie auch mechanischen Kontakt zwischen jeweils einer elektrischen Zu- und Ableitung 3 und der keramikadapterplattenseitig angebrachten elektrischen Leiterstruktur 12 zu verbessern, können in an sich bekannter Weise zwei, drei oder mehrere derartiger Mikroflexkontakte 13 nebeneinander längs der innerhalb des Trägersubstrats 4 verlaufenden elektrischen Zu- und Ableitung 3 vorgesehen werden.

Selbstverständlich sind die gesamten in den Figuren 2b und 2c dargestellten, elektrischen Verbindungsanordnungen mit einem biokompatiblen Kunststoff möglichst vollständig fluiddicht umfasst.

Es liegt auf der Hand, dass der erforderliche Bauraum für die bekannte elektrische Verbindungsstruktur mit zunehmender Komplexität und Multipolarität der zu implantierenden Einheit zunimmt, wodurch auch die Patientenbelastung und -irritation in gleicher Weise zunimmt.

Der Druckschrift US 5,324,322 A ist eine implantierbare Dünnfilm-Elektrodenanordnung zu entnehmen, die ein einheitliches Dünnfilmflächensubstrat aufweist, das einen zu einer Wickelelektrode verformbaren ersten Abschnitt, einen zweiten Abschnitt zur elektrischen Kontaktierung mit einer elektrischen Zu- und Ableitungsstruktur sowie einen beide Abschnitte miteinander verbindenden dritten Abschnitt aufweist. Über die Ausgestaltung der Kontaktenden seitens der elektrischen Zu- und Ableitungsstruktur wird in dieser Druckschrift nichts ausgeführt.

### Darstellung der Erfindung

Der Erfindung liegt die Aufgabe zugrunde eine implantierbare elektrische Verbindungsstruktur zwischen einem elektrischen Implantat, das über wenigstens einen elektrischen Leiter verfügt, und einer elektrischen Zu- und Ableitungsstruktur,
derart weiterzubilden, dass die patientenseitige Belastung gegenüber bekannten, gattungsgemäßen Verbindungsstrukturen signifikant reduziert werden soll. So soll insbesondere eine möglichst flexible Verbindungsstruktur geschaffen werden, die über ein hohes Maß an Zuverlässigkeit in Bezug auf eine dauerhafte elektrische Verbindung gewährleisten soll.

Die Lösung der der Erfindung zugrundeliegenden Aufgabe ist im Anspruch 1 angegeben. Gegenstand des Anspruches 8 ist ein lösungsgemäßes Verfahren zur Herstellung der Verbindungsstruktur. Den Erfindungsgedanken in vorteilhafter Weise weiterbildende Merkmale sind Gegenstand der Unteransprüche sowie der weiteren Beschreibung, insbesondere unter Bezugnahme auf die Ausführungsbeispiele zu entnehmen.

Die lösungsgemäße implantierbare elektrische Verbindungsstruktur zwischen einem elektrischen Implantat, das über wenigstens einen elektrischen Leiter verfügt, und einer elektrischen Zu- und Ableitungsstruktur zeichnet sich dadurch aus, dass die elektrische Zu- und Ableitungsstruktur wenigstens ein elektrisches Kabel mit einem Kabelende umfasst, an dem freitragend ein elektrisch leitendes Flächenstück angebracht ist, an bzw. auf das der wenigstens eine implantatseitige elektrische Leiter unmittelbar gefügt ist.

Die lösungsgemäße Verbindungsstruktur vermeidet somit die starre und flächig ausgebildete Keramikadapterplatte und eröffnet auf diese Weise die Möglichkeit einer hochflexiblen Ausgestaltung der implantierbaren elektrischen Verbindungsstruktur mit einer damit verbundenen miniaturisierbaren Bauweise, durch die eine mit der Implantation verbundene patientenspezifische Belastung signifikant reduziert werden kann.

Der Erfindung liegt die Idee zugrunde, jeweils das Ende eines implantatseitigen elektrischen Leiters mit dem Kabelende eines Kabels, das Teil einer elektrischen Zu- und Ableitungsstruktur ist und bspw. mit einem elektrischen Energie- und/oder Steuermodul verbunden ist, unmittelbar elektrisch leitend miteinander zu verbinden, wobei die elektrische Verbindung robust und mechanisch belastbar ausgebildet ist, so dass sie Druck- und Zugkräften standhält, die von elastischen oder plastischen Verformungen des unmittelbar an die Verbindung angrenzenden Kabelabschnittes bzw. des implantatseitigen elektrischen Leiters herrühren können. Um den hohen Grad nach Flexibilität zu schaffen, ist das jeweils am Kabelende angebrachte Flächenstück ohne jegliche mechanische Unterstützung freitragend angebracht und definiert den elektrischen und mechanischen Verbindungsbereich jeweils zwischen einem implantatseitigen elektrischen Leiter und einem Kabel.

Das vorzugsweise aus dem gleichen metallischen Material bestehende Flächenstück, aus dem das Kabel selbst gefertigt ist, kann in Form einer separaten Komponente ausgebildet sein, die mittels einer Fügeverbindung am Kabelende unlösbar fest angebracht ist, bspw. im Wege einer Löt-, Schweiß-, Klebe- oder mechanischen Klemmverbindung.

Die Größe und Form des vorzugsweise als metallisches Plättchen ausgebildeten Flächenstückes ist aus Gründen einer möglichst miniaturisierten Ausbildung der implantierbaren, elektrischen Verbindungsstruktur ausschließlich zu Zwecken einer mechanisch festen und elektrisch leitenden Verbindung zwischen jeweils einem Kabelende der elektrisch Zu- und Ableitungsstruktur und einem implantatseitigen elektrischen Leiter gewählt und misst lediglich Flächengrößen im Bereich weniger µm².

Gleichsam bietet es sich an, das Flächenstück mittels einer Materialumformung des Kabelmaterials am Kabelende auszubilden, so dass das Flächenstück einstückig mit dem jeweiligen Kabel verbunden ist.

Das aus einem flexiblen, folienartigen, elektrisch nicht leitendem Flächenelement bestehende Trägersubstrat verleiht der Vielzahl der in dem folienartigen Flächenelement eingebetteten elektrischen Leiter sowohl eine definierte relative Anordnung zueinander sowie auch einen mechanischen Halt zu deren gesamtheitlichen Handhabung. In einer besonders bevorzugten Ausführungsform weist das folienartige Trägersubstrat zum Zwecke der elektrischen Kontaktierung der einzelnen elektrischen Leiter in einem randseitigen Folienbereich fingerartig ausgebildete Folienendabschnitte auf, längs der jeweils wenigstens ein elektrischer Leiter eingebettet ist. In jedem der fingerartigen Folienendabschnitte ist wenigstens eine die Folie samt den wenigstens einen elektrischen Leiter durchsetzende Öffnung eingebracht, innerhalb der der elektrische Leiter über eine frei zugängliche Leiteroberfläche verfügt, die im Wege einer Schweiß-, Klebe-, Drahtbond-, Ballbond- oder Lötverbindung jeweils mit einem Flächenstück eines Kabels elektrisch und mechanisch stabil verbunden wird.

Zum Fügen jeweils eines an einem Kabelende angebrachten Flächenstückes an die in den fingerartigen Folienendabschnitten bereichsweise offenliegenden elektrischen Leiterenden bedarf es lediglich einer gegenseitigen räumlichen Überdeckung und der Fügung, vorzugsweise unter Ausbildung eines lokalen Mikroflexkontaktes. Um den elektrischen und mechanischen Kontakt zwischen einem elektrischen Leiter und einem Flächenstück zu verbessern können mehrere Mikroflexkontakte längs eines Leiterendes vorgesehen sein.

Durch die räumlich getrennte Ausbildung der elektrischen Kontakte zwischen den Kabeln und den elektrischen Leitern, d.h. zu jedem fingerartigen Folienendabschnitt führt vorzugsweise nur ein Kabel, können sowohl die Kabel unabhängig voneinander räumlich angeordnet werden, bspw. um sie zu einem möglichst schlanken Kabelstrang zusammenzuführen, als auch die fingerartigen Folienendabschnitte im Rahmen der Folienflexibilität zusammengewickelt oder -gerollt werden, um so eine möglichst kompakte bzw. platzsparende Foliengeometrie zu bilden.

Die lösungsgemäße implantierbare elektrische Verbindungsstruktur lässt sich in einer besonders vorteilhaften Weise herstellen. So gilt es zunächst das wenigstens eine, der elektrischen Zu- und Ableitungsstruktur zugehörige Kabel mit einem am Kabelende angebrachten elektrisch leitfähigen Flächenstückes bereitzustellen.

Das Bereitstellen eines derart vorkonfektionierten Kabels kann entweder durch Fügen eines separaten metallischen Flächenstückes an ein Kabelende oder durch mechanisches Umformen eines Kabelendes unter Ausbildung eines einstückig mit dem Kabelende verbundenen Flächenstückes erfolgen.

Im Weiteren wird jeweils das am Kabelende angebrachte Flächenstück mit einem frei zugänglichen Endabschnittes eines implantatseitigen elektrischen Leiters gefügt. Die Handhabung des Kabels und des elektrischen Leiters während des Fügevorganges, bei dem vorzugsweise eine Schweiß-, Klebe-, Drahtbond-, Ballbond- oder Lötverbindungstechnik zum Einsatz kommt, vereinfacht sich durch den mechanisch stabilen Verbund des wenigstens einen elektrischen Leiters innerhalb des flexiblen, folienartigen, elektrisch nicht leitenden Flächenelementes, das ortsfest auf einer Unterlage positioniert wird. So bedarf es lediglich einer Positionierung des kabelseitigen Flächenstückes am Ort des freiliegenden Leiterendabschnittes vorzugsweise zwischen Unterlage und Flächenstück. Bspw. durch Aufbringen eines Gold-Ballbondes im Bereich der fingerartigen Folienendabschnitte samt den elektrischen Leiterendabschnitten durchsetzenden Öffnungen wird ein stabiler Mikroflexkontakt hergestellt.

Die nachfolgenden Figuren illustrieren eine bevorzugte Ausführungsform einer lösungsgemäß ausgebildeten implantierbaren elektrischen Verbindungsstruktur. Am Schluss wird vorzugsweise noch ein polymeres Verkapselungsmaterial um die elektrische Verbindungsstruktur angebracht, um die elektrische Verbindung vor dem wässrigen Milieu des Körpers zu schützen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a: Illustration der lösungsgemäßen implantierbaren elektrischen Verbindungsstruktur,
- Fig. 1b: Längsschnitt durch einen Mikroflexkontakt, sowie
- Fig. 2a, b, c: an sich bekannte implantierbare Verbindungsstruktur gemäß Stand der Technik.

### Wege zur Ausführung der Erfindung, gewerbliche Verwendbarkeit

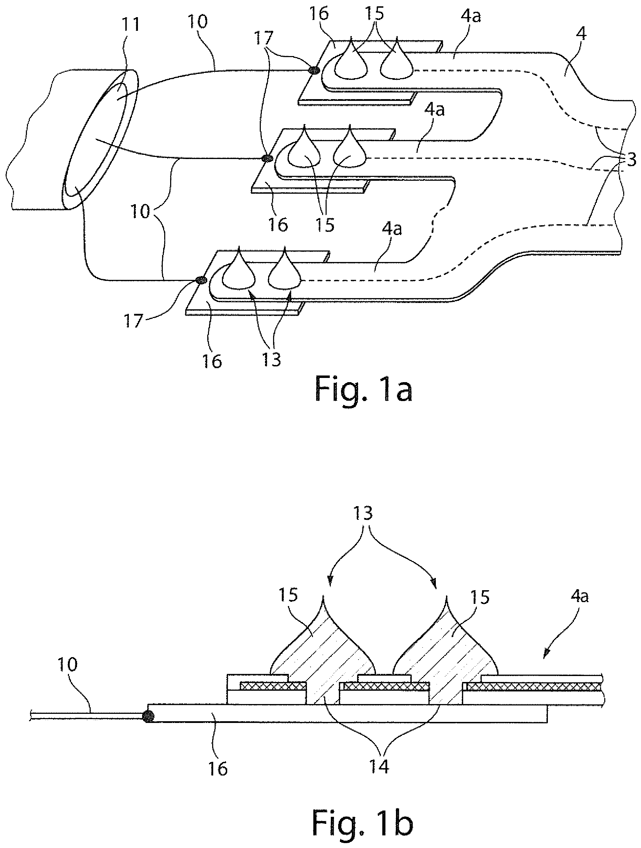

Figur 1a zeigt eine lösungsgemäße implantierbare elektrische Verbindungsstruktur zwischen einer Anzahl zu einem Implantat I führenden elektrischen Leitern 3 und einer entsprechenden Anzahl von elektrischen Versorgungskabel 10, die zu einer elektrischen Zu- und Ableitungsstruktur 11 zusammengefasst sind und bspw. mit einem Energie- und Steuermodul verbunden sind.

Die implantatseitig vorgesehenen elektrischen Leiter 3 sind in üblicher Weise in einem Trägersubstrat 4 in Form eines flexiblen, folienartig ausgebildeten, elektrisch nicht leitenden Flächenelementes 4 integriert, vorzugsweise in Form einer biokompatiblen Polymerfolie. Jede einzelne elektrische Leitung 3 mündet im gezeigten Ausführungsbeispiel längs eines fingerartig ausgebildeten Folienendabschnittes 4a. Die Anzahl der einzelnen Folienendabschnitt 4a entspricht der Anzahl der einzelnen zum Implantat I führenden elektrischen Leitungen 3.

Zur Kontaktierung der elektrischen Leitungen 3 mit jeweils einem elektrischen Versorgungskabel 10 sieht jedes Kabel 10 ein metallisches, plättchenförmiges Flächenstück 16 vor, das entweder über eine Fügeverbindung 17 mit dem Ende eines Kabels 10 verbunden ist, bspw. mittels Schweiß-, Klebe-, Drahtbond- oder Lötverbindung, oder einstückig im Wege einer Materialumformung aus dem Kabelende geformt ist, siehe auch Längsschnittdarstellung in Figur 1b durch einen Folienendabschnitt 4a mit zwei Mikroflexkontakten 13 .

Jedes der fingerartig ausgebildeten Folienendabschnitte 4a weist zur Ausbildung zweier Mikroflexkontakte 13 zwei Öffnungen 14, die sowohl den Folienendabschnitt 4a als auch den elektrischen Leiter 3 lokal durchsetzen. Jeder einzelne Folienendabschnitt 4a liegt einseitig unmittelbar auf einer Oberfläche des Flächenstückes 16 auf, dessen Dicke einige 10 bis einige 100 µm betragen kann. Zur elektrischen und mechanischen festen Fügung werden die jeweils zwei Öffnungen 14 innerhalb jedes einzelnen Folienendabschnittes 4a mit einem Ballbond 15, vorzugsweise einem Goldbond verfüllt.

Bedarfsweise kann auch nur ein Mikroflexkontakt 13 oder es können mehr Mikroflexkontakte 13 längs eines elektrischen Leiters 3 vorgesehen sein, je nach Maßgabe der zu erwartenden Belastungssituation, die die Verbindungsstruktur Stand zu halten hat.

Die Form und Flächengröße der einzelnen Flächenstücke 16 sind jeweils in Hinblick auf eine möglichst miniaturisierte und kompakte Bauform jeder einzelnen elektrischen Verbindungsstruktur zu wählen und richtet sich vornehmlich an die Form und Größe der implantatseitigen Folienendabschnitte 4a.

Selbst bei einer großen Anzahl von elektrischen Leitern 3 und den mit diesen verbundenen Kabeln 10 ist es möglich die fingerartigen Folienendabschnitte 4a durch Wickeln bspw. um eine in Längserstreckung der Folienendabschnitte 4a orientierten Achse in eine kleine zylinderförmige Bauform überzuführen, von der die Kabel 10 eng aneinander liegend in die elektrische Zu- und Ableitungsstruktur 11 einmünden. Ebendiese Verformbarkeit der lösungsgemäß ausgebildeten implantierbaren elektrischen Verbindungsstruktur macht den besonderen Vorteil aus, durch den die Miniaturisierbarkeit der Struktur ermöglicht wird.

Selbstverständlich sind auch andere Formen und Geometrien für die Ausgestaltung des Kontaktbereiches des folienartigen Trägersubstrats 4a denkbar. Auch für die Ausgestaltung der elektrischen und mechanischen Fügeverbindung zwischen den einzelnen elektrischen Leitern 3 und den Flächenstücken 16 der Kabel 10 eignen sich grundsätzlich alle dem Fachmann bekannten und für diesen konkreten Anwendungsfall geeigneten Verbindungstechniken, wie bspw. das Reibschweißen, Ultraschallschweißen, Löten, Kleben, Bondverfahren etc..

Um zu vermeiden, dass die elektrisch leitenden Flächenstücke 16 elektrische Kurzschlüsse bilden, gilt es diese vor einer platzsparenden Formung der Verbindungsstruktur mit einem elektrisch isolierenden Schichtmaterial oder einer Vergussmasse zu umschließen.

### Bezugszeichenliste

- 1: Cuff-Elektrodenanordnung
- 2: Nervenfaserbündel
- 3: Elektrische Leitungen
- 4: Biokompatibles Trägersubstrat, Polymerfolie
- 4a: Folienendabschnitt
- 5: Mikroflexstrukturen
- 6: Keramikadapterplatte
- 7: Mikroflexkontakte, Mikroflexpads
- 8: Elektrodenflächen
- 9: Lötstelle
- 10: Kabel
- 11: Kabelstrang, elektrischen Zu- und Ableitungsstruktur
- 12: elektrische Leiterstruktur
- 13: Mikroflexkontakt
- 14: Öffnung
- 15: Ballbond, Goldbond
- 16: Flächenstück
- 17: Fügestelle

## Patentansprüche

1. Implantierbare elektrische Verbindungsstruktur zwischen einem elektrischen Implantat, das über wenigstens einen elektrischen Leiter (3) verfügt, und einer elektrischen Zu- und Ableitungsstruktur, wobei die elektrische Zu- und Ableitungsstruktur wenigstens ein elektrisches Kabel (10) mit einem Kabelende umfasst,
**dadurch gekennzeichnet, dass** an dem Kabelende freitragend, d.h. ohne mechanische Stützstruktur, ein elektrisch leitendes Flächenstück (16) angebracht ist, und
dass an das Flächenstück (16) der wenigstens eine Implantat-seitige elektrische Leiter (3) gefügt ist.

2. Implantierbare elektrische Verbindungsstruktur nach Anspruch 1,
**dadurch gekennzeichnet, dass** das Flächenstück (16) in Form einer zum Kabel (10) separaten Komponente an das Kabelende gefügt ist

3. Implantierbare elektrische Verbindungsstruktur nach Anspruch 1 oder 2,
**dadurch gekennzeichnet, dass** das Flächenstück (16) in Art eines metallischen Plättchens ausgebildet ist.

4. Implantierbare elektrische Verbindungsstruktur nach einem der Ansprüche 1 bis 3,
**dadurch gekennzeichnet, dass** die Fügung zwischen dem Flächenstück (16) und dem wenigstens einen Implantat-seitigen elektrischen Leiter (3) eine Schweiß-, Klebe-, Drahtbond-, Ballbond- oder Lötverbindung darstellt.

5. Implantierbare elektrische Verbindungsstruktur nach Anspruch 1,
**dadurch gekennzeichnet, dass** das Flächenstück (16) mittels Materialverformung des Kabelmaterials am Kabelende erhalten ist, das einstückig mit dem Kabel (10) verbunden ist.

6. Implantierbare elektrische Verbindungsstruktur nach einem der Ansprüche 1 bis 5,
**dadurch gekennzeichnet, dass** der wenigstens eine Implantat-seitige elektrische Leiter (3) in einem flexiblen, folienartigen, elektrisch nicht leitenden Flächenelement (4) integriert und mit dem elektrischen Implantat elektrisch verbunden ist, und
dass der wenigstens eine elektrische Leiter (3) einen frei zugänglichen Leiterendabschnitt aufweist, der mit dem Flächenstück (16) eines Kabels (10) elektrisch verbunden ist.

7. Implantierbare elektrische Verbindungsstruktur nach einem der Ansprüche 1 bis 6,
**dadurch gekennzeichnet, dass** der wenigstens eine Implantat-seitige elektrische Leiter (3) unmittelbar auf dem Flächenstück (16) gefügt ist.

8. Verfahren zur Herstellung einer implantierbaren elektrischen Verbindung zwischen einem elektrischen Implantat, das über wenigstens einen elektrischen Leiter (3) verfügt, und einer elektrischen Zu- und Ableitungsstruktur nach einem der Ansprüche 1 bis 7, **gekennzeichnet durch** folgende Verfahrensschritte:
- Bereitstellen eines der elektrischen Zu- und Ableitungsstruktur zugehörigen Kabels (10) mit einem Kabelende, an dem ein freitragendes, d.h. ohne mechanische Stützstruktur, elektrisch leitendes Flächenstück (16) angebracht ist,
- Fügen des elektrischen Leiters (3) an das elektrisch leitende Flächenstück (16).

9. Verfahren nach Anspruch 8,
**dadurch gekennzeichnet, dass** das Flächenstück (16) im Wege einer Fügung an das Kabelende des Kabels (10) angebracht wird.

10. Verfahren nach Anspruch 8,
**dadurch gekennzeichnet, dass** das Flächenstück (16) im Wege einer einstückigen Materialumformung des Kabels (10) am Kabelende hergestellt wird.

11. Verfahren nach einem der Ansprüche 8 bis 10,
**dadurch gekennzeichnet, dass** der wenigstens eine in einem flexiblen, folienartigen, elektrisch nicht leitenden Flächenelement (16) integrierte und mit dem elektrischen Implantat elektrisch verbundene elektrische Leiter (3) mit seinem frei zugänglichen Leiterendabschnitt unmittelbar an das Flächenstück (16) derart gefügt wird, so dass das mit dem Flächenstück (16) verbundene Kabel (10) relativ zum Flächenelement verformbar ist.

12. Verfahren nach Anspruch 11,
**dadurch gekennzeichnet, dass** das wenigstens eine Kabel (10) zusammen mit dem an das Flächenstück (16) gefügten frei zugänglichen Leiterendabschnitt mit einer elektrisch isolierenden Vergussmasse oder einer elektrisch isolierenden Schutzhülle umfasst werden.

## Claims

1. Implantable electrical connecting structure between an electrical implant that comprises at least one electrical conductor (3), and an electrical feed and drain line structure, wherein the electrical feed and drain line structure comprises at least one cable (10) with a cable end,
**characterised in that** applied on the cable end in a self-supporting manner, i.e. without a mechanical supporting structure, is an electrically conducting planar piece (16), and
**in that** the at least one electrical conductor (3) on the implant side is joined to the planar piece (16).

2. Implantable electrical connecting structure according to claim 1,
**characterised in that** the planar piece (16) is joined to the cable end in the form of a component separate to the cable (10).

3. Implantable electrical connecting structure according to claim 1 or 2, **characterised in that** the planar piece (16) is designed in the form of a of small metallic plate.

4. Implantable electrical connecting structure according to any one of claims 1 to 3, **characterised in that** the joint between the planar piece (16) and the at least one electrical conductor (3) on the implant side is a welded, adhesive, wire bond, ball bond or soldered connection.

5. Implantable electrical connecting structure according to claim 1, **characterised in that** the planar piece (16) is obtained by means of material deformation of the cable material at the cable end that is connected in one piece to the cable (10).

6. Implantable electrical connection structure according to any one of claims 1 to 5, **characterised in that** the at least one electrical conductor (3) on the implant side is integrated into a flexible, foil-like, electrically non-conducting planar piece (4) and electrically connected to the electrical implant, and **in that** the at least one electrical conductor (3) has a freely accessible conductor end section which is electrically connected with the planar piece (16) of a cable (10).

7. Implantable electrical connecting structure according to any one of claims 1 to 6, **characterised in that** the at least one electrical conductor (3) on the implant side is directly joined to the planar piece (16).

8. Method of manufacturing an implantable electrical connection between an electrical implant that has at least one electrical conductor (3), and an electrical feed and drain line structure according to any one of claims 1 to 7**characterised by** the following process stages:
- provision of a cable (10) belonging to an electrical feed and drain line, with a cable end to which a self-supporting, i.e. without a mechanical support structure, electrically conducting planar piece (16) is applied,
- joining of the electrical conductor (3) to the electrically conducting planar piece (16).

9. Method according to claim 8, **characterised in that** the planar piece (16) is applied to the cable end of the cable (10) by way of a joint.

10. Method according to claim 8, **characterised in that** the planar piece (16) is produced by way of one-piece material reshaping of the cable (10) at the cable end.

11. Method according to any one of claims 8 to 10, **characterised in that** the at least one electrical conductor (3), which is integrated into a flexible, foil-like, electrically non-conductive planar piece (16) and is electrically connected to the electrical implant, is joined with its freely accessible conductor end section to the planar piece (16) in such a way that the cable (10) connected to the planar piece (16) can be reshaped relative to the planar piece.

12. Method according to claim 11, **characterised in that** the at least one cable (10) together with the freely accessible conductor end section joined to the planar piece (16) are surrounded by an electrically insulating casting compound or an electrically insulating protective sleeve.

## Revendications

1. Structure de connexion électrique implantable entre un implant électrique, qui dispose d'au moins un conducteur électrique (3), et une structure d'alimentation et de dérivation électrique, sachant que la structure d'alimentation et de dérivation électrique comprend au moins un câble électrique (10) avec une extrémité de câble,
**caractérisée en ce qu'**un élément de surface (16) électroconducteur est placé en saillie à l'extrémité de câble, c'est-à-dire sans structure de support mécanique, et
**en ce qu'**au moins un conducteur électrique (3) côté implant est adapté à l'élément de surface (16).

2. Structure de connexion électrique implantable selon la revendication 1,
**caractérisée en ce que** l'élément de surface (16) est adapté au câble (10) à l'extrémité de câble sous la forme d'un composant séparé.

3. Structure de connexion électrique implantable selon la revendication 1 ou 2, **caractérisée en ce que** l'élément de surface (16) est constitué sous la forme d'une plaquette métallique.

4. Structure de connexion électrique implantable selon l'une quelconque des revendications 1 à 3, **caractérisée en ce que** le montage entre l'élément de surface (16) et au moins un conducteur électrique côté implant (3) représente une liaison par soudure, par collage, par câblage filaire, par soudure en boule ou par brasage.

5. Structure de connexion électrique implantable selon la revendication 1, **caractérisée en ce que** l'élément de surface (16) est obtenu au moyen de la déformation de matériau du matériau de câble à l'extrémité de câble, qui est reliée en une pièce au câble (10).

6. Structure de connexion électrique implantable selon l'une quelconque des revendications 1 à 5, **caractérisée en ce qu'**au moins un conducteur électrique côté implant (3) est intégré dans un élément de surface flexible, de type feuille, non électroconducteur (4) et est relié électriquement à l'implant électrique, et **en ce qu'**au moins un conducteur électrique (3) comporte une section de conducteur librement accessible, qui est reliée électriquement à l'élément de surface (16) d'un câble (10) .

7. Structure de connexion électrique implantable selon l'une quelconque des revendications 1 à 6, **caractérisée en ce qu'**au moins un conducteur électrique (3) du côté implant est adapté directement à la pièce de surface (16) .

8. Procédé de fabrication d'une structure de connexion électrique implantable entre un implant électrique, qui dispose d'au moins un conducteur électrique (3) et une structure d'alimentation et de dérivation électrique selon l'une quelconque des revendications 1 à 7, **caractérisé par** les étapes de procédé suivantes :
- préparation d'un câble (10) appartenant à la structure d'alimentation et de dérivation électrique avec une extrémité de câble à laquelle est placée un élément de surface (16) électroconducteur en saillie, c'est-à-dire sans structure de support mécanique,
- montage du conducteur électrique (3) sur l'élément de surface électroconducteur (16).

9. Procédé selon la revendication 8, **caractérisé en ce que** l'élément de surface (16) est placé au cours d'un montage à l'extrémité de câble du câble (10).

10. Procédé selon la revendication 8, **caractérisé en ce que** l'élément de surface (16) est réalisé à l'extrémité de câble au cours d'une déformation de matériau en une pièce du câble (10).

11. Procédé selon l'une quelconque des revendications 8 à 10, **caractérisé en ce qu'**au moins le conducteur électrique (3) intégré dans un élément de surface (16) flexible, en forme de feuille, non électroconducteur et relié à l'implant électrique est adapté directement à l'élément de surface (16) avec sa section finale de conducteur librement accessible de telle manière que le câble (10) relié à l'élément de surface (16) peut être déformé par rapport à l'élément de surface.

12. Procédé selon la revendication 11, **caractérisé en ce qu'**au moins un câble (10) avec la section finale de conducteur facilement accessible adaptée à l'élément de surface (16) sont entourés d'une masse de remplissage électro-isolante ou d'une gaine de protection électro-isolante.
